# DEMANDE DE BREVET EUROPEEN

(11) **EP 3 260 530 A1**
(43) Date de publication de la demande: **27.12.2017**
(21) Numéro de dépôt: 17176167.9
(22) Date de dépôt: 15.06.2017
(51) Int. Cl.: C12M 1/00

(54) **SYSTÈME ET PROCÉDÉ POUR LYSER UN ÉCHANTILLON BIOLOGIQUE**

(30) Priorité: 20.06.2016 FR 1655703
(71) Demandeur: COMMISSARIAT À L'ÉNERGIE ATOMIQUE ET AUX ÉNERGIES ALTERNATIVES, 75015 Paris (FR)
(72) Inventeur: LAMOTTE, Hadrien, 38190 VILLARD BONNOT (FR); ACHARD, Jean-Luc, 38100 GRENOBLE (FR); ROUX, Jean-Maxime, 38700 LA TRONCHE (FR)
(74) Mandataire: GIE Innovation Competence Group

(57) **Abrégé**

L'invention concerne un procédé mis en oeuvre pour lyser des espèces biologiques présentes dans une solution aqueuse et pour récupérer un matériel biologique issu de la lyse des espèces biologiques, ledit procédé étant mis en oeuvre en employant un système qui comporte :
- Une cuve (20) délimitant un volume,
- Une première électrode (21) et une deuxième électrode (22) comprenant chacune une extrémité placée dans ledit volume formé par la cuve,
- Un circuit générateur d'impulsions de tension (3).

Ledit procédé consiste à appliquer au moins une impulsion de tension entre les deux électrodes à l'aide du circuit générateur d'impulsions de tension (3) connecté aux deux électrodes, pour entraîner la création d'un arc électrique entre l'extrémité de la première électrode et l'extrémité de la deuxième électrode et la génération d'au moins une onde de choc dans la solution.

## Description

### Domaine technique de l'invention

La présente invention se rapporte à un procédé de lyse d'espèces biologiques et de récupération du matériel biologique issu de la lyse et à un système pour lyser un échantillon biologique et pour récupérer le matériel biologique issu de la lyse des espèces biologiques.

### Etat de la technique

La détection de pathogènes dans un échantillon biologique est souvent réalisée en employant un matériel lourd et peu adapté. La détection nécessite notamment une étape préalable de lyse des espèces biologiques contenues dans l'échantillon pour en dégager un matériel biologique à analyser.

Il existe plusieurs méthodes pour lyser des espèces biologiques. A titre d'exemple, celles-ci peuvent être de type :
- Chimique, en ajoutant des réactifs dans la solution qui contient les espèces biologiques à lyser.
- Mécanique, par broyage, par des ondes de choc produites par Laser ou par des ondes de choc produites par ultrasons.
- Thermique.
- Electrique, par électroporation.

La demande de brevet WO2015/181743A1 décrit notamment un mécanisme de lyse mécanique par broyage. Dans celui-ci, la lyse mécanique est plus particulièrement réalisée par cisaillement entre deux parois, l'une des deux parois ayant une surface d'appui rugueuse.

Les demandes de brevet WO2007/061943A2, US2012/219987A1 décrivent des solutions de lyse par électroporation.

Les différentes solutions connues présentent toutes certains avantages mais elles ont aussi un certain nombre d'inconvénients listés ci-dessous :
- La méthode chimique requiert l'apport de réactifs dans la solution, ce qui a tendance à polluer la solution.
- La méthode mécanique par broyage est difficile à mettre en oeuvre de manière automatique.
- La méthode par ondes de choc produites par un Laser nécessite un matériel coûteux.
- La méthode par ondes de choc par ultrasons est difficile à mettre en oeuvre et génère du bruit.
- La méthode thermique peut entraîner une dénaturation des espèces biologiques.
- La méthode électrique par électroporation, telle que décrite dans les documents antérieurs précités, ne présente pas un rendement très satisfaisant.

Un premier but de l'invention est donc de proposer un procédé de lyse d'espèces biologiques contenues dans une solution et de récupération du matériel biologique obtenu à l'issue de la lyse, ce procédé étant fiable, facile à mettre en oeuvre, peu couteux, ne nécessitant pas une concentration en espèces biologiques élevée et facilement automatisable.

### Exposé de l'invention

Ce but est atteint par un procédé mis en oeuvre pour lyser des espèces biologiques présentes dans une solution aqueuse et pour récupérer un matériel biologique issu de la lyse des espèces biologiques, ledit procédé étant mis en oeuvre à l'aide d'un système pour lyser lesdites espèces biologiques, ledit système comprenant :
- Une cuve délimitant un volume,
- Une première électrode comprenant une extrémité placée dans ledit volume formé par la cuve,
- Une deuxième électrode comprenant une extrémité placée dans ledit volume formé par la cuve et séparée de l'extrémité de la première électrode d'une distance d'écartement non nulle,
- L'extrémité de la première électrode et l'extrémité de la deuxième électrode étant configurées et agencées pour assurer entre elles un effet de pointe en présence d'un champ électrique,
- Un circuit générateur d'impulsions de tension,

Ledit procédé comportant les étapes suivantes :
- Remplissage au moins partiel du volume de la cuve avec la solution contenant les espèces biologiques à lyser de manière à ce que l'extrémité de la première électrode et l'extrémité de la deuxième électrode soient plongées dans ladite solution,
- Application d'au moins une impulsion de tension entre les deux électrodes à l'aide du circuit générateur d'impulsions de tension connecté aux deux électrodes, ladite impulsion étant générée à une amplitude suffisante et pendant une durée suffisante pour entraîner la création d'un arc électrique entre l'extrémité de la première électrode et l'extrémité de la deuxième électrode, entraînant la génération d'au moins une onde de choc dans ladite solution,
- Récupération du matériel biologique issu de la lyse des espèces biologiques contenues dans la solution.

Les solutions décrites dans l'état de la technique précité ne permettent pas la création d'un arc électrique, la configuration des électrodes n'étant pas adaptée pour cela. Dans ces documents, les électrodes sont en effet agencées pour présenter des surfaces en vis-à-vis entre lesquelles est formée le champ électrique nécessaire à la mise en oeuvre de l'électroporation.

Selon une particularité du procédé, lors de l'étape de remplissage, la cuve est remplie partiellement par la solution contenant les espèces à lyser, de manière à laisser un volume d'air entre la surface de la solution et le haut de la cuve, permettant ainsi la création d'une bulle de gaz après la génération de ladite onde de choc.

Selon une autre particularité, lors de l'étape de remplissage, le volume de la cuve est rempli en totalité et le procédé comporte une étape d'arrêt du remplissage par capillarité.

Selon une autre particularité, l'étape de récupération dudit matériel biologique comporte une étape ultérieure d'éjection de la solution en dehors de la cuve, causée par la génération de ladite onde de choc.

Selon un mode de réalisation particulier, l'étape de remplissage est mise en oeuvre par le principe des vases communicants.

Un autre but est également de proposer un nouveau système pour lyser des espèces biologiques contenues dans une solution, qui permette de palier les inconvénients de l'art antérieur. Le système de l'invention est notamment :
- Peu encombrant,
- Facile à mettre en oeuvre, car automatique,
- D'un coût modéré,
- Non polluant pour la solution,
- D'un rendement satisfaisant,
- D'un coût énergétique assez faible,
- Ne nécessitant pas une forte concentration en espèces biologiques.

L'invention concerne également un système pour lyser un échantillon biologique selon le procédé défini ci-dessus, ledit système comportant :
- Une cuve délimitant un volume destiné à être rempli au moins partiellement par une solution contenant des espèces biologiques à lyser,
- Une première électrode comprenant une extrémité placée dans ledit volume formé par la cuve,
- Une deuxième électrode comprenant une extrémité placée dans ledit volume formé par la cuve et séparée de l'extrémité de la première électrode d'une distance d'écartement non nulle,
- L'extrémité de la première électrode et l'extrémité de la deuxième électrode étant configurées et agencées pour assurer entre elles un effet de pointe en présence d'un champ électrique,
- Un circuit électrique générateur d'impulsions relié à la première électrode et à la deuxième électrode.

Préférentiellement, ladite plus grande dimension définissant la surface transversale de chaque électrode en contact avec la solution est par exemple inférieure à 50% de ladite distance d'écartement.

Avantageusement, l'extrémité de la première électrode et l'extrémité de la deuxième électrode sont chacune en forme de pointe.

Avantageusement, chaque pointe est formée suivant un axe et l'axe suivi par l'extrémité de la première électrode et l'axe suivi par l'extrémité de la deuxième électrode sont confondus.

Préférentiellement, le circuit générateur d'impulsions comporte :
- Un générateur haute tension présentant deux bornes d'alimentation connectées chacune à une électrode distincte,
- Un condensateur connecté en parallèle des deux électrodes aux deux bornes du générateur par l'intermédiaire d'une résistance,
- Un interrupteur pour commander la décharge du condensateur et commander ainsi le déclenchement des impulsions de tension entre les deux électrodes.

Avantageusement, le générateur haute tension est agencé pour fournir une tension comprise entre 5 et 15 kV.

Selon un mode de réalisation particulier, le système comporte au moins un réflecteur acoustique, positionné pour réfléchir chaque onde de choc généré vers l'intérieur du volume de la cuve.

Selon un mode de réalisation particulier, la cuve définit un volume présentant un fond et un goulot définissant un orifice d'éjection.

Avantageusement, le système comporte des conduits de remplissage reliés à la cuve pour alimenter la cuve en solution contenant des espèces biologiques à lyser selon le principe des vases communicants.

Avantageusement, lesdits conduits de remplissage sont agencés pour déboucher dans le fond de la cuve.

Avantageusement, l'orifice d'éjection du goulot est dimensionné pour créer une vanne capillaire stoppant le remplissage de la cuve lorsque la cuve est remplie jusqu'au niveau de son goulot.

### Brève description des figures

D'autres caractéristiques et avantages vont apparaître dans la description détaillée qui suit faite en regard des dessins annexés dans lesquels :
- La figure 1 représente, de manière générale, la structure du système de l'invention, permettant la mise en oeuvre du procédé de l'invention.
- La figure 2 représente un mode de réalisation préféré du circuit générateur d'impulsions qui est employé.
- La figure 3 représente un mode de réalisation particulier du système de l'invention.
- Les figures 4A à 4C représentent des variantes de réalisation du système de l'invention employant des réflecteurs ou un agencement particulier de la cuve du système de l'invention.
- La figure 5 illustre les différentes étapes du procédé de l'invention, mises en oeuvre pour la lyse des espèces biologiques à l'aide du système de l'invention.
- La figure 6 représente une variante de réalisation du système de l'invention, permettant la lyse des espèces biologiques contenues dans la solution et une récupération de la solution lysée.
- La figure 7 représente une courbe reliant la hauteur de solution dans le réservoir contenant la réserve de solution à lyser au rayon de l'orifice d'éjection présent sur la cuve.
- La figure 8 représente les différentes étapes du procédé de lyse et d'extraction mises en oeuvre à l'aide du système représenté sur la figure 6.
- La figure 9 illustre un exemple de mise en oeuvre du procédé de l'invention.

### Description détaillée d'au moins un mode de réalisation

Le système de l'invention est destiné à permettre une lyse des espèces biologiques contenues dans un échantillon afin d'en libérer un matériel biologique à étudier. Cet échantillon biologique se présente par exemple sous la forme d'une solution aqueuse qui contient les espèces biologiques. Par espèces biologiques, on entend notamment des micro-organismes, des cellules, des spores, des bactéries, des micro-algues, des champignons, des virus... Par matériel biologique à étudier, on entend par exemple des molécules d'acide nucléique (ARN, ADN) issues d'une cellule, des protéines, des lipopolysaccharides (LPS), des acides lipotéichoïques (LTA)...

Dans la suite de la description, de manière non limitative, nous prendrons comme exemple un échantillon biologique comprenant des espèces biologiques présentes dans une solution telle que l'eau.

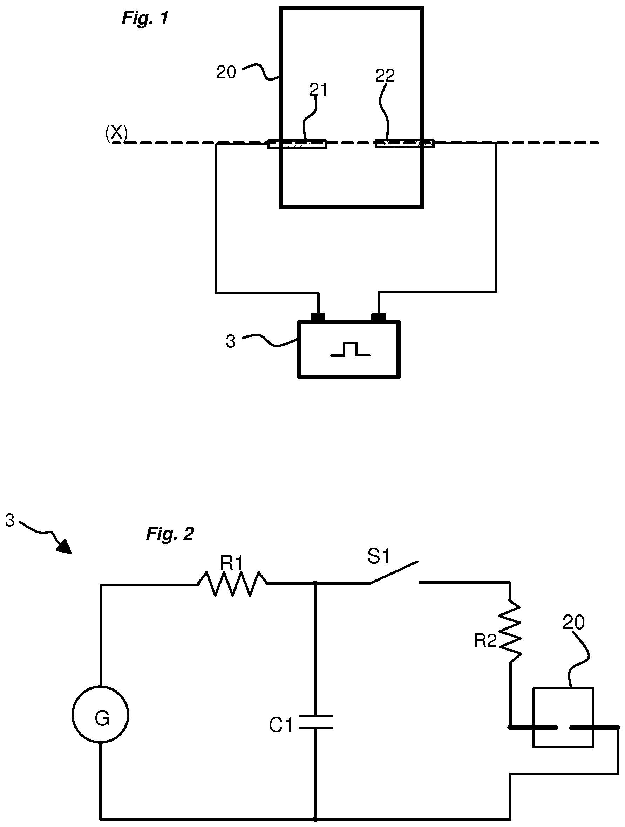

Selon l'invention, la lyse est mise en oeuvre par effet électrique. En référence à la figure 1, le système de l'invention comporte ainsi :
- Une cuve 20 qui définit un volume destiné à recevoir la solution qui contient les espèces biologiques à lyser.
- Une première électrode 21 présentant une extrémité placée dans le volume de la cuve 20.
- Une deuxième électrode 22 présentant une extrémité placée dans le volume de la cuve, l'extrémité de la deuxième électrode 22 étant positionnée par rapport à l'extrémité de la première électrode à une distance d'écartement e déterminée, non nulle.
- Un circuit électrique générateur d'impulsions 3 de tension, relié à la première électrode 21 et à la deuxième électrode 22.

Selon l'invention, on considérera que la distance d'écartement e évoquée dans la présente description est la distance la plus courte qui sépare les deux extrémités des électrodes 21, 22 plongées dans la solution.

Selon l'invention, chaque électrode 21, 22 se termine par au moins une partie allongée s'étendant selon un axe longitudinal.

Chaque électrode est configurée et agencée pour assurer à son extrémité un effet de pointe (connu dans le domaine des paratonnerres) par rapport à l'autre lorsque des impulsions de tension sont appliquées entre les deux électrodes. En effet, la pointe de ces objets amplifie le champ électrique en leur voisinage.

Selon l'invention, l'extrémité de la première électrode 21 qui est en contact avec la solution 4 à lyser et l'extrémité de la deuxième électrode 22 qui est en contact avec la solution à lyser sont chacune formées d'une surface transversale, configurées pour assurer un effet de pointe et permettre entre elles la génération d'un arc électrique.

La surface transversale de chaque électrode est avantageusement configurée en dimensions et position par rapport à la surface transversale de l'autre électrode pour assurer la génération de l'arc électrique entre ces deux surfaces lors de l'application d'impulsions de tension d'un niveau déterminé.

La surface transversale de chaque électrode présente une plus grande dimension qui est faible par rapport à la distance d'écartement e séparant les deux extrémités des électrodes. La distance d'écartement e est préférentiellement comprise entre 1 et 4mm. Ladite plus grande dimension de la surface transversale sera préférentiellement inférieure à 50% de ladite distance d'écartement e.

Avantageusement, chaque électrode 21, 22 se termine à son extrémité par une pointe, formant ladite surface transversale de création de l'arc électrique. Les deux pointes sont situées en vis-à-vis l'une de l'autre. Les deux pointes sont par exemple de forme rectiligne et réalisées chacune selon ledit axe longitudinal. Avantageusement, l'axe suivi par la pointe de la première électrode et l'axe suivi par la pointe de la deuxième électrode sont confondus (voir axe (X) sur les figures). En amont de sa pointe, chaque électrode pourra prendre une forme quelconque.

Par ailleurs, il faut noter que l'arc électrique créé entre les extrémités des deux électrodes 21, 22 sera parfaitement localisé dans la solution et sera toujours et uniquement généré dans une même zone située entre les deux pointes et non de manière aléatoire à l'intérieur de la cuve 20 contenant la solution. Cette architecture permet ainsi une reproductibilité dans l'application de la lyse. La zone de génération de l'arc électrique est définie par un volume délimité dans la solution à lyser (forcément inférieur au volume total de la cuve), autour du segment rectiligne joignant les extrémités des deux électrodes 21, 22. L'arc électrique créé débute à l'extrémité de l'une des deux électrodes et se termine à l'extrémité de l'autre électrode.

A partir de ce système, l'invention consiste à créer une ou plusieurs ondes de choc dans la solution 4 aqueuse qui contient les échantillons biologiques, en vue de fragiliser et/ou détruire des parois des espèces biologiques présentes dans la solution 4 et ainsi de libérer le matériel biologique.

Pour cela, le circuit électrique générateur d'impulsions 3 génère des impulsions de tension au sein de la solution 4 contenant les espèces biologiques à lyser, conduisant au claquage de la solution, c'est-à-dire à l'apparition d'un arc électrique entre les extrémités des deux électrodes 21, 22. Lors de l'apparition de cet arc électrique, une première série d'ondes de choc comprenant au moins une onde de choc est générée dans la solution 4, permettant de lyser les espèces biologiques et ainsi de libérer le matériel biologique.

Selon la configuration, suit alors l'apparition d'une bulle de gaz (principalement de la vapeur d'eau) dans la zone de la solution où l'arc électrique s'est formé. Cette bulle de gaz croît jusqu'à sa taille maximale puis s'effondre rapidement, générant alors une deuxième série d'ondes de choc. Par ailleurs, dans certaines conditions et pour certaines espèces biologiques à lyser, on pourra observer également la création d'un nuage de cavitation dans la solution au-dessus des électrodes, formées de plusieurs bulles de cavitation engendrées par des dépressions associées à chaque série d'ondes de choc. Chacune des bulles du nuage de cavitation s'effondre ensuite ce qui génère localement de multiples ondes de chocs.

Bien entendu, on comprend que pour obtenir l'effet escompté, différents paramètres électriques devront être réglés de manière adaptée aux conditions de fonctionnement. Il s'agira en effet d'appliquer au moins une impulsion de tension d'amplitude et de durée suffisante pour entraîner la création de l'arc électrique entre les extrémités des deux électrodes 21, 22 plongées dans la solution. Ces paramètres de l'impulsion de tension incluent de surcroît :
- La distance d'écartement e entre les deux extrémités des électrodes 21, 22, celle-ci devant permettre la création de l'arc électrique lorsqu'une impulsion de tension est générée.
- La conductivité de la solution 4 dans laquelle sont placées les espèces biologiques, celle-ci ayant une influence sur la génération de l'arc électrique entre les deux électrodes 21, 22 et influera donc forcément sur la distance d'écartement e entre les deux extrémités des électrodes 21, 22.

Par ailleurs, en vue d'optimiser le système de l'invention, il s'agira également de veiller à la durée du front montant de l'impulsion et à la durée du front descendant de l'impulsion. De manière non limitative, le front montant aura une durée inférieure à 1 microseconde, préférentiellement inférieure à 500 nanosecondes.

Avantageusement, afin de permettre la création de la bulle de gaz après la première série d'ondes de choc, il est nécessaire de tenir compte des paramètres suivants :
- La hauteur *hsol* de solution au-dessus des électrodes, celle-ci devant être adaptée pour permettre une croissance suffisante de la bulle de gaz dans la solution après la formation du canal de vapeur. Celle-ci sera à adapter en fonction des espèces biologiques à lyser. Par exemple, si les espèces biologiques sont des micro-algues, la hauteur *hsol* de solution au-dessus des électrodes doit être suffisante pour permettre le développement d'un nuage de cavitation (c'est-à-dire le développement de micro-bulles suite au passage de la première série d'ondes de choc) au-dessus des électrodes. Si les espèces biologiques à lyser sont des bactéries végétatives, la hauteur *hsol* de solution doit être suffisante pour permettre le développement de la bulle de gaz entre les électrodes, mais rester relativement faible pour permettre de confiner la deuxième série d'ondes de choc engendrée par l'effondrement de la bulle de gaz.
- La hauteur *hair* d'air au-dessus de la surface de la solution, celle-ci influant sur la taille de la bulle de gaz générée.
- La section d'ouverture du volume de la cuve sur sa partie supérieure, par exemple définie par sa longueur L et sa largeur l si la section d'ouverture est rectangulaire, ou son diamètre si la section est circulaire

A partir de ces différents paramètres, à titre d'exemple et de manière non limitative, on effectue une lyse dans les conditions décrites ci-dessous.

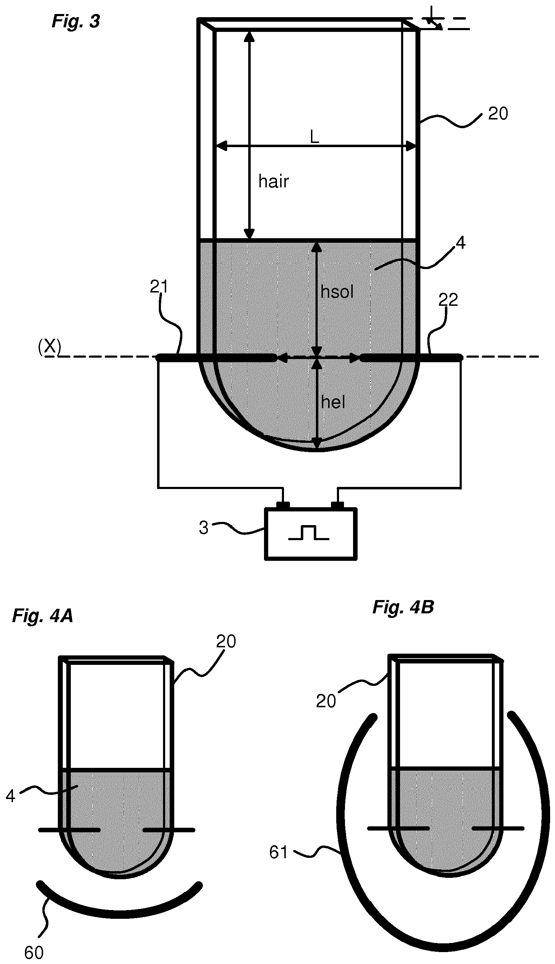

En référence à la figure 3, la cuve 20 présente par exemple une section longitudinale définissant un volume en forme de U et une section d'ouverture dans le plan transversal qui est de forme rectangulaire. La cuve 20 sera par exemple fabriquée par toute technique classique connue, telle que par exemple usinage, moulage, impression... Elle sera par exemple obtenue par usinage dans un matériau plastique (par exemple de type Topas (marque déposée) 5013) ou par impression par stéréolithographie dans un polymère (par exemple de type Perfactory (marque déposée) Envision LS600). La cuve 20 sera préférentiellement fermée de manière étanche à son sommet, par un couvercle adapté.

Comme décrit ci-dessus, les deux électrodes 21, 22 se terminent par exemple chacune par une pointe droite à leur extrémité. Les deux pointes ainsi formées sont par exemple placées l'une en face de l'autre dans le volume de la cuve, suivant un même axe (X) et distantes l'une de l'autre d'une distance d'écartement e adaptée.

En référence à la figure 2, le circuit générateur d'impulsions 3 comporte par exemple :
- Un générateur haute tension G présentant deux bornes d'alimentation connectées chacune à une électrode 21, 22 distincte.
- Un condensateur C1 connecté en parallèle des deux électrodes aux deux bornes du générateur G par l'intermédiaire d'une résistance R1.
- Un interrupteur S1 pour commander la décharge du condensateur C1 et commander ainsi le déclenchement des impulsions de tension entre les deux électrodes 21, 22.
- Une résistance de protection R2 placée en série avec les électrodes 21, 22 pour éviter tout risque de court-circuit du condensateur C1.

En outre, selon les espèces biologiques à lyser et l'application visée, en référence à la figure 3, on respectera les conditions opérationnelles définies dans l'un des deux tableaux présentés ci-dessous.

Pour l'analyse/identification (par exemple de bactéries, champignons, virus, ou micro-algues) :

| ***Paramètre*** | ***Définition*** | ***Valeurs*** |
|---|---|---|
| ***hₛₒₗ*** | *Hauteur de solution au- dessus des électrodes* | *Comprise entre 4 mm et 10 mm - de préférence 7 mm* |
| ***hₐᵢᵣ*** | *Hauteur d'air présente au- dessus de la surface de la solution* | *Comprise entre 2 mm et 10 mm - de préférence 5 mm* |
| ***hₑₗ*** | *Hauteur des électrodes par rapport au fond du volume de la cuve* | *Comprise entre 3 mm et 6 mm - de préférence 3 mm* |
| ***L*** | *Longueur de la section d'ouverture de la cuve* | *Comprise entre 6 mm et 10 mm - de préférence 6 mm* |
| ***l*** | *Largeur de la section d'ouverture de la cuve* | *Comprise entre 1 mm et 4 mm - de préférence 2 mm* |
| ***e*** | *Distance d'écartement entre les pointes des électrodes* | *Comprise entre 1 mm et 4 mm - de préférence 2 mm* |

Pour l'extraction de composés d'intérêt (par exemple le contenu de micro-algues), afin d'observer la génération d'un nuage de cavitation au-dessus des électrodes, il faudra respecter les conditions opérationnelles suivantes :

| ***Paramètre*** | ***Définition*** | ***Valeurs*** |
|---|---|---|
| ***hₛₒₗ*** | *Hauteur de solution au- dessus des électrodes* | *Comprise entre 10 mm et 100 mm - de préférence 30 mm* |
| ***hₐᵢᵣ*** | *Hauteur d'air présente au- dessus de la surface de la solution* | *Comprise entre 2 mm et 10 mm - de préférence 5 mm* |
| ***h**ₑₗ* | *Hauteur des électrodes par rapport au fond du volume de la cuve* | *Comprise entre 3 mm et 10 mm - de préférence 3 mm* |
| ***L*** | *Longueur de la section d'ouverture de la cuve* | *Comprise entre 6 mm et 20 mm - de préférence 6 mm* |
| ***l*** | *Largeur de la section d'ouverture de la cuve* | *Comprise entre 1 mm et 5 mm - de préférence 2 mm* |
| ***e*** | *Distance d'écartement entre les pointes des électrodes* | *Comprise entre 1 mm et 5 mm - de préférence 2 mm* |

Pour générer les ondes de choc dans de telles conditions de fonctionnement, le générateur de tension G est par exemple capable de fournir une tension de plusieurs kilovolts, par exemple comprise entre 5 et 15 kV, préférentiellement environ 10 kV, sous la forme d'impulsions présentant chacune une durée de quelques dizaines de microsecondes chacune, comprise entre 20 et 40µs, préférentiellement d'environ 30µs. Ces paramètres de fonctionnement du générateur de tension G doivent cependant être adaptés à la conductivité de la solution contenant les espèces biologiques présente dans la cuve 20. Ils seront notamment suffisants pour permettre le claquage d'une solution 4 ayant une conductivité peu élevée, typiquement comprise entre 30 et 500 S.m⁻¹. Lors du claquage de la solution, le générateur délivre un pic de courant d'une très forte intensité pouvant aller jusqu'à 10⁴ A.

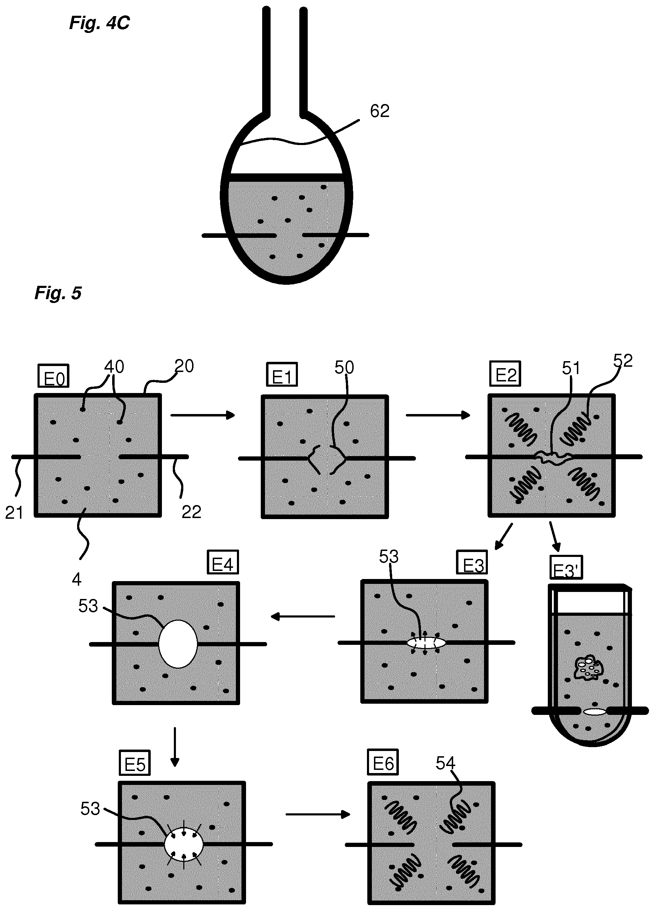

En référence à la figure 5, dans de telles conditions, le principe de lyse mis en oeuvre dans le procédé de l'invention est le suivant :
- La solution 4 qui contient des espèces biologiques 40 à lyser est placée dans la cuve 20 du système de l'invention (étape E0).
- Le circuit générateur d'impulsions (non représenté sur la figure 5) génère une impulsion à haute tension conformément aux caractéristiques définies ci-dessus, générant des arcs électriques 50 entre les deux électrodes (étape E1).
- Les arcs électriques générés créent un canal conducteur 51 entre les deux électrodes, entraînant le claquage de la solution (étape E2).
- Le claquage de la solution entraîne la génération d'au moins une première série d'ondes de choc 52 (étape E2) entraînant une première lyse en permettant une fragilisation et/ou une destruction des parois des espèces biologiques 40 présentes dans la solution 4.
- A l'issue de l'onde de choc, une bulle de vapeur 53 se crée entre les deux électrodes (étape E3), favorisée par l'espace libre présent dans la cuve au-dessus de la surface de la solution. Dans certaines conditions, la première série d'ondes de choc pourra entraîner la génération de bulles de cavitation au-dessus des électrodes (étape E3').
- La bulle de vapeur 53 générée croît progressivement jusqu'à une taille maximale (étape E4).
- Après avoir atteint sa taille maximale, la bulle de vapeur 53 implose (étape E5). De même chaque bulle de cavitation générée au-dessus des électrodes implose (non représentée).
- Lors de l'implosion de la bulle de vapeur 53 et des bulles de cavitation, une deuxième série d'ondes de choc 54 est générée, entraînant une deuxième lyse des espèces biologiques 40 présentes dans la solution 4 (étape E6).
- De nouvelles bulles de cavitation peuvent apparaître à l'issue de la deuxième série d'ondes de choc. En implosant, celles-ci entraîneront la naissance de nouvelles ondes de chocs locales.

Le caractère automatisable du principe de lyse décrit ci-dessus permet au procédé de l'invention de permettre aisément une récupération du matériel biologique issu de la lyse des espèces biologiques.

Le principe est ici d'employer la génération des ondes de chocs pour éjecter au moins une partie du volume de la solution présente dans la cuve 20 et ainsi de récupérer facilement une solution qui contient des espèces biologiques lysées sans aucune intervention humaine.

Par ailleurs, pour optimiser la mise en oeuvre de ce procédé d'extraction, deux principes décrits ci-dessous peuvent être pris en combinaison.

Le premier principe est celui des vases communicants. En effet, on prévoit de remplir la cuve 20 avec la solution qui contient les espèces biologiques à lyser en employant le principe des vases communicants. Le système de l'invention comporte ainsi au moins un conduit de remplissage qui débouche dans la partie inférieure de la cuve 20, préférentiellement au fond de celle-ci, et qui est alimenté par un réservoir de solution, contenant les espèces biologiques à lyser et positionné au-dessus du sommet de la cuve 20, à une hauteur adaptée pour entraîner un remplissage de la cuve par le principe des vases communicants.

Le second principe employé utilise la capillarité. Il s'agit de mettre en oeuvre une vanne capillaire pour stopper naturellement le remplissage de la cuve 20 lorsque celle-ci est pleine. Pour cela, la cuve 20 comporte dans sa partie supérieure un goulot définissant un orifice d'éjection de la solution. Cet orifice présente un diamètre adapté pour stopper le remplissage de la cuve par la solution qui contient les espèces biologiques à lyser lorsque le volume de la cuve est plein et que la surface de la solution atteint la section la plus étroite du goulot.

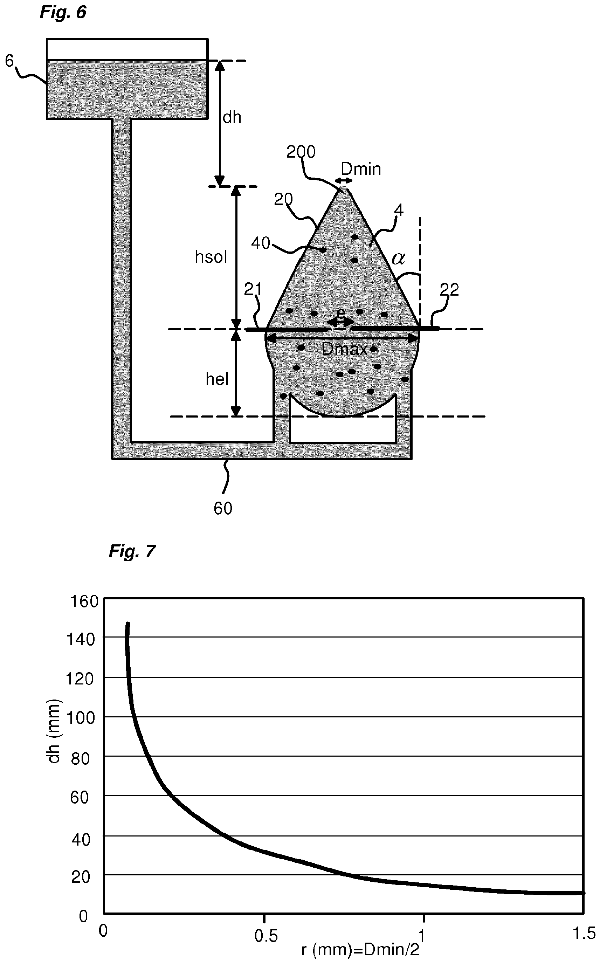

La figure 6 représente ainsi le système amélioré pour lyser les espèces biologiques contenues dans la solution et récupérer la solution lysée, ce système agencé notamment pour mettre en oeuvre les deux principes décrits ci-dessus.

Sur cette figure 6, la cuve 20 se présente par exemple sous la forme d'une bonbonne comportant une partie inférieure dont le fond est arrondi et une partie supérieure de forme conique et dont le sommet est percé d'un orifice 200 axial. Cet orifice 200 axial est dimensionné pour permettre la mise en oeuvre du principe de vanne capillaire décrit ci-dessus.

Comme décrit ci-dessus, le système comporte par exemple un conduit de remplissage 60 alimenté par un réservoir 6 et débouchant en deux points distincts dans la partie inférieure de la cuve 20, préférentiellement sous les électrodes et donc dans le fond de la cuve.

Comme décrit ci-dessus, le réservoir 6 est rempli d'une solution comportant des espèces biologiques à lyser. Pour mettre en oeuvre le principe des vases communicants, ce réservoir doit forcément être positionné au-dessus du sommet de la cuve pour pouvoir la remplir en totalité.

Par ailleurs, pour mettre en oeuvre le principe de vanne capillaire, il sera nécessaire d'assurer une pression suffisante de liquide au niveau de l'orifice 200 d'éjection. La figure 7 montre ainsi une courbe indiquant une relation entre la différence de hauteur (dh), entre la surface de solution dans le réservoir 6 et le sommet de la cuve 20, et le rayon (r) de l'orifice 200 d'éjection présent sur la cuve 20 (également défini par le diamètre Dmin sur la figure 6).

Les électrodes 21, 22 sont positionnés comme décrit précédemment et le circuit générateur d'impulsions 3 de tension reste identique.

En référence à la figure 6, pour mettre en oeuvre le procédé d'une manière fiable et efficace, le système employé sera préférentiellement dimensionné de la manière suivante :

| ***Paramètre*** | ***Définition*** | ***Valeurs*** |
|---|---|---|
| ***hₛₒₗ*** | *Hauteur de solution au- dessus des électrodes* | *Comprise entre 1 mm et 8 mm - de préférence 3 mm* |
| ***hₑₗ*** | *Hauteur des électrodes par rapport au fond du volume de la cuve* | *Comprise entre 3 mm et 10 mm - de préférence 4 mm* |
| ***Dmax*** | *Diamètre maximal de la cuve au niveau de la base de son cône* | *Compris entre 5 et 10 mm - de préférence 6 mm* |
| ***Dmin*** | *Diamètre de l'orifice d'éjection* | *Compris entre 0.2 mm et 1.2 mm - de préférence entre 0.5 et 0.8 mm* |
| ***α*** | *Angle définissant la partie conique de la bobonne* | *Compris entre 20 et 50 ° - de préférence 30°* |
| ***e*** | *Distance d'écartement entre les pointes des électrodes* | *Comprise entre 1 mm et 4 mm - de préférence 2 mm* |

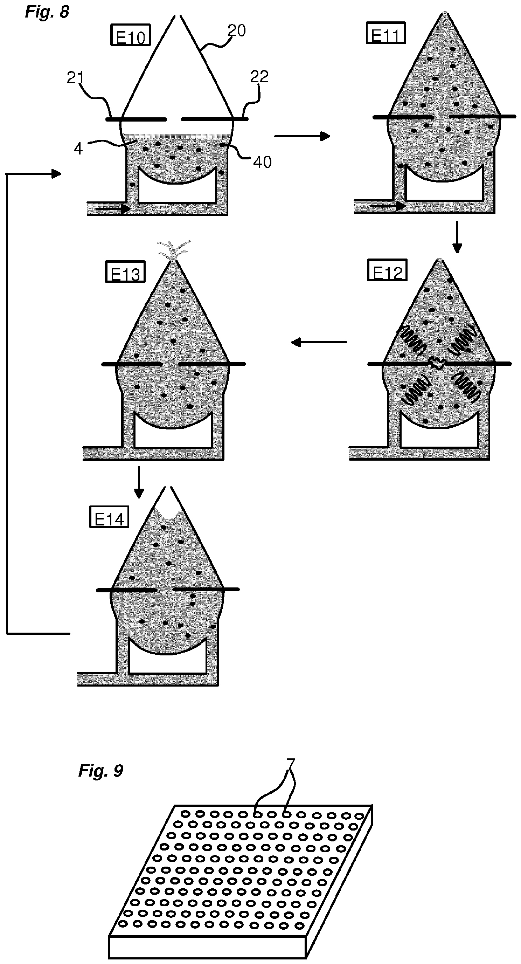

La figure 8 illustre les différentes étapes mises en oeuvre à l'aide du système décrit ci-dessus et représenté sur la figure 6.
- Par le principe des vases communicants, la cuve 20 est remplie à partir du réservoir en solution contenant des espèces biologiques à lyser - étape E10.
- La vanne capillaire au sommet de la cuve stoppe le remplissage de la cuve lorsque la cuve est remplie - étape E11.
- Comme décrit ci-dessus, on procède ensuite aux étapes de lyse des espèces biologiques contenues dans la solution. Il s'agit donc de générer une première série d'ondes de choc par claquage de la solution en appliquant des impulsions de tension. Ce principe est décrit ci-dessus en liaison avec la figure 5 - étape E12.
- La génération de la première série d'ondes de choc entraîne l'éclatement de la vanne capillaire et donc l'éjection d'une partie de la solution lysée en dehors de la cuve. Cet échantillon de solution lysée peut ainsi être aisément récupérée, sans avoir à manipuler la cuve. Cette étape se produit simultanément à l'étape précédente de génération de la première série d'ondes de choc- étape E13.
- Une fois, l'éjection terminée, le remplissage de la cuve 20 peut reprendre comme à l'étape E10.

Le procédé décrit ci-dessus est notamment efficace pour lyser des espèces biologiques comme des micro-algues. Ces micro-algues seront par exemple de type Nannochloropsis ou Phaeodactylum, dont on extrait des lipides ou de la chlorophylle.

La fréquence des impulsions émises par le circuit générateur d'impulsions 3 de tension est adaptée au rythme de remplissage de la cuve. Pour éjecter un maximum de solution lysée en dehors de la cuve 20, il est nécessaire d'attendre que la cuve soit remplie en totalité. Un détecteur de niveau de solution dans la cuve pourra être prévu pour s'assurer que la cuve 20 est bien remplie lorsque le circuit générateur d'impulsions 3 de tension est commandé. Un automate programmable sera parfaitement adapté pour mettre en oeuvre une telle séquence de fonctionnement.

Par ailleurs, comme représenté sur la figure 9, pour rentabiliser au maximum le procédé, on pourra installer en parallèle plusieurs unités 7 de lyse et de récupération, chacune sous la forme d'un système tel que décrit ci-dessus en liaison avec la figure 6, par exemple avec un réservoir 6 commun à toutes les unités. Chaque unité sera en mesure de produire un volume déterminé de solution lysée. En employant un système présentant les caractéristiques décrites ci-dessus, chaque unité pourra par exemple produire un volume de 5 microlitres par seconde. En parallélisant une centaine d'unités, il est ainsi possible d'obtenir un volume de solution lysée de 16 litres par heure, en fonctionnant à une fréquence de deux impulsions haute tension par seconde.

Selon l'invention, en référence aux figures 4A à 4C, afin d'améliorer l'efficacité du système de l'invention et de mieux profiter de chaque onde de choc généré, il est possible de mettre en oeuvre une réflexion des ondes de choc. Pour cela, il s'agit par exemple d'ajouter un ou plusieurs réflecteurs acoustiques de forme adaptée autour du volume défini par la cuve 20 pour focaliser les ondes de chocs à l'intérieur de la cuve. Les figures 4A à 4C présentent ainsi quelques modes de réalisation distincts.

Sur la figure 4A, on utilise un réflecteur 60 partiel en arc de cercle positionné sous le fond de la cuve.

Sur la figure 4B, un réflecteur 61 de forme ovale vient englober tout le volume défini par la cuve.

Sur la figure 4C, le volume interne défini par la cuve est en forme d'ellipse formant lui-même un réflecteur 62 acoustique pour les ondes de choc générés.

La solution de l'invention décrite ci-dessus, présente ainsi de nombreux avantages, notamment :
- Elle est simple à mettre en oeuvre car ne nécessite pas de matériel complexe, ni coûteux.
- Elle peut être mise en oeuvre de manière automatique car il s'agit simplement de commander le circuit générateur d'impulsions.
- Elle n'entraîne aucune pollution de la solution,
- Elle permet de récupérer aisément de la solution lysée, sans avoir à manipuler la cuve.
- Même traitée en petite quantité, la fréquence de génération des impulsions permet de lyser et de récupérer une quantité importante de solution, la rendant ainsi exploitable à plus grande échelle.

## Revendications

1. Procédé mis en oeuvre pour lyser des espèces biologiques présentes dans une solution aqueuse et pour récupérer un matériel biologique issu de la lyse des espèces biologiques, ledit procédé étant mis en oeuvre à l'aide d'un système pour lyser lesdites espèces biologiques, ledit système comprenant :
- Une cuve (20) délimitant un volume,
- Une première électrode (21) comprenant une extrémité placée dans ledit volume formé par la cuve,
- Une deuxième électrode (22) comprenant une extrémité placée dans ledit volume formé par la cuve et séparée de l'extrémité de la première électrode d'une distance d'écartement non nulle,
- L'extrémité de la première électrode (21) et l'extrémité de la deuxième électrode (22) étant configurées et agencées pour assurer entre elles un effet de pointe en présence d'un champ électrique,
- Un circuit générateur d'impulsions de tension (3),
Ledit procédé étant **caractérisé en ce qu'**il comporte les étapes suivantes :
- Remplissage au moins partiel du volume de la cuve (20) avec la solution (4) contenant les espèces biologiques (40) à lyser de manière à ce que l'extrémité de la première électrode (21) et l'extrémité de la deuxième électrode (22) soient plongées dans ladite solution,
- Application d'au moins une impulsion de tension entre les deux électrodes à l'aide du circuit générateur d'impulsions de tension (3) connecté aux deux électrodes, ladite impulsion étant générée à une amplitude suffisante et pendant une durée suffisante pour entraîner la création d'un arc électrique entre l'extrémité de la première électrode et l'extrémité de la deuxième électrode, entraînant la génération d'au moins une onde de choc dans ladite solution,
- Récupération du matériel biologique issu de la lyse des espèces biologiques contenues dans la solution.

2. Procédé selon la revendication 1, **caractérisé en ce que** lors de l'étape de remplissage, la cuve (20) est remplie partiellement par la solution contenant les espèces à lyser, de manière à laisser un volume d'air entre la surface de la solution (4) et le haut de la cuve, permettant ainsi la création d'une bulle de gaz après la génération de ladite onde de choc.

3. Procédé selon la revendication 1, **caractérisé en ce que** lors de l'étape de remplissage, le volume de la cuve est rempli en totalité et **en ce qu'**il comporte une étape d'arrêt du remplissage par capillarité.

4. Procédé selon la revendication 3, **caractérisé en ce que** l'étape de récupération dudit matériel biologique comporte une étape ultérieure d'éjection de la solution en dehors de la cuve, causée par la génération de ladite onde de choc.

5. Procédé selon la revendication 3 ou 4, **caractérisé en ce que** l'étape de remplissage est mise en oeuvre par le principe des vases communicants.

6. Système pour lyser un échantillon biologique selon le procédé défini dans l'une des revendications 1 à 5, **caractérisé en ce qu'**il comporte :
- Une cuve (20) délimitant un volume destiné à être rempli au moins partiellement par une solution (4) contenant des espèces biologiques (40) à lyser,
- Une première électrode (21) comprenant une extrémité placée dans ledit volume formé par la cuve,
- Une deuxième électrode (22) comprenant une extrémité placée dans ledit volume formé par la cuve et séparée de l'extrémité de la première électrode d'une distance d'écartement (e) non nulle,
- L'extrémité de la première électrode (21) et l'extrémité de la deuxième électrode (22) étant configurées et agencées pour assurer entre elles un effet de pointe en présence d'un champ électrique,
- Un circuit électrique générateur d'impulsions (3) relié à la première électrode et à la deuxième électrode.

7. Système selon la revendication 6, **caractérisé en ce que** ladite plus grande dimension définissant la surface transversale de chaque électrode en contact avec la solution est par exemple inférieure à 50% de ladite distance d'écartement.

8. Système selon la revendication 6 ou 7, **caractérisé en ce que** l'extrémité de la première électrode et l'extrémité de la deuxième électrode sont chacune en forme de pointe.

9. Système selon la revendication 8, **caractérisé en ce que** chaque pointe est formée suivant un axe et **en ce que** l'axe suivi par l'extrémité de la première électrode et l'axe suivi par l'extrémité de la deuxième électrode sont confondus.

10. Système selon l'une des revendications 6 à 9, **caractérisé en ce que** le circuit générateur d'impulsions comporte :
- Un générateur haute tension (G) présentant deux bornes d'alimentation connectées chacune à une électrode distincte,
- Un condensateur (C1) connecté en parallèle des deux électrodes aux deux bornes du générateur par l'intermédiaire d'une résistance (R1),
- Un interrupteur (S1) pour commander la décharge du condensateur et commander ainsi le déclenchement des impulsions de tension entre les deux électrodes.

11. Système selon la revendication 10, **caractérisé en ce que** le générateur haute tension (G) est agencé pour fournir une tension comprise entre 5 et 15 kV.

12. Système selon l'une des revendications 6 à 11, **caractérisé en ce qu'**il comporte au moins un réflecteur (60, 61, 62) acoustique, positionné pour réfléchir chaque onde de choc généré vers l'intérieur du volume de la cuve.

13. Système selon l'une des revendications 6 à 12, **caractérisé en ce que** la cuve définit un volume présentant un fond et un goulot définissant un orifice (200) d'éjection.

14. Système selon la revendication 13, **caractérisé en ce qu'**il comporte des conduits de remplissage (60) reliés à la cuve (20) pour alimenter la cuve (20) en solution (4) contenant des espèces biologiques à lyser selon le principe des vases communicants.

15. Système selon la revendication 14, **caractérisé en ce que** lesdits conduits de remplissage (60) sont agencés pour déboucher dans le fond de la cuve (20).

16. Système selon l'une des revendications 13 à 15, **caractérisé en ce que** l'orifice (200) d'éjection du goulot est dimensionné pour créer une vanne capillaire stoppant le remplissage de la cuve (20) lorsque la cuve (20) est remplie jusqu'au niveau de son goulot.
